(19) Europäisches Patentamt
European Patent Office
Office européen des brevets

(11) **EP 0 308 493 B1**

## (12) EUROPEAN PATENT SPECIFICATION

(45) Date of publication and mention
of the grant of the patent:
**28.05.1997 Bulletin 1997/22**

(21) Application number: **88903639.8**

(22) Date of filing: **31.03.1988**

(51) Int Cl.6: **G01N 23/00**, G01B 15/02

(86) International application number:
**PCT/US88/00975**

(87) International publication number:
**WO 88/07671 (06.10.1988 Gazette 1988/22)**

(54) **THICKNESS/DENSITY MEASURING APPARATUS**

GERÄT ZUR MESSUNG DER DICKE ODER DES SPEZIFISCHEN GEWICHTES

APPAREIL DE MESURE D'EPAISSEUR/DENSITE

(84) Designated Contracting States:
**BE DE FR GB IT NL SE**

(30) Priority: **31.03.1987 US 32639**

(43) Date of publication of application:
**29.03.1989 Bulletin 1989/13**

(73) Proprietor: **ADAPTIVE TECHNOLOGIES INC.**
**Gaithersburg, Maryland 20879 (US)**

(72) Inventor: **PETTIT, John W.**
**Derwood, MD 20855 (US)**

(74) Representative: **Daley, Michael John et al**
**F.J. CLEVELAND & COMPANY**
**40-43 Chancery Lane**
**London WC2A 1JQ (GB)**

(56) References cited:
**US-A- 3 016 460**       **US-A- 3 562 500**
**US-A- 4 292 645**       **US-A- 4 301 366**
**US-A- 4 427 892**

- **JOURNAL OF APPLIED PHYSICS, vol. 53, no. 4, April 1982, pages 2975-2982, American Institute of Physics, New York, US; P.D. PEDROW et al.: "Detection of x rays from the anode of a vacuum arc"**

## Description

### Background of the Invention

#### 1. Field of the Invention

The present invention relates to a thickness or density measuring apparatus which is particularly adapted to measure the thickness or density of a film. The invention has particular, though not exclusive, utility in the plastics processing industry where thickness and/or density of an extruded plastic film must be controlled to production tolerances.

#### 2. Description of the Prior Art

Thickness and/or density measuring systems are known in the art which rely on various types of sensors for a measurement. For thickness measurements, mechanical gauge-type devices are known which contact with the material or object to be measured and which provide a thickness readout which can be used to control apparatus for manufacturing the material and/or object to insure that a desire thickness is obtained. Likewise, density measuring devices are known in which the density of an object is measured usually by passing radiation through the object. Changes in density are seen by changes in the transmittance of the radiation through the object.

In many production environments particularly where plastic films and sheets are being manufactured, the control over material usage and quality of the product is greatly enhanced when the thickness of the material can be accurately measured. This is especially true when the measurement can be made in an on-line manner where the measurement instrument can be installed on or quite close to the producing machinery and the thickness information can be used in a feedback control system for the process so that optimum parameters can be determined for the machinery to produce a desired film product. In such an environment the output of the measuring instrument must respond quickly to thickness variations, and the output is fed back electronically or mechanically to control machinery without any human intervention. In order to do this, the instrument must be inherently rugged to withstand the harshness of the production environment, be small enough in size to be able to fit where the measurement is most needed, and have minimal input and output connections so that integration with computing and control equipment can be facilitated.

At present, many thickness measurements are made using the principles of detection of nuclear and atomic particle radiation emitted from a source. U.S. Patent No. 4,047,029 is representative and discusses various radiation sources including those producing beta particles, X-rays and gamma rays. These rays are attenuated or scattered when they pass through a material as a consequence of their interaction with the atoms and nuclei in the material. The amount of interaction is dependent on the number of atoms and nuclei in the path of the radiation and the tendency of the material to interact with the type of radiation striking it. For a given material and type of radiation the amount of interaction will depend on the material's density and thickness since density determines the number of atoms and nuclei in the path of the radiation per unit volume and thickness determines the length of the travel when the radiation is made to pass through the material, usually at right angles to a flat surface.

Present generation thickness measuring devices based on the principles of particle detection will detect and count individually particles that pass through a sample of a material and compare the count rate observed for an unknown sample with a count rate observed with a sample of known thickness. The material thickness is then inferred by assuming similar density between the known and unknown samples and applying, typically, a linear relationship between the count rate and material thickness. The relationship is typically not linear over a very large range but can usually be assumed to be linear over the range of thicknesses encountered in a production environment, without significant loss of accuracy.

The method currently employed for detecting such radiation works through the use of a scintillation detector or a gas filled ionization counter. The ionization counter consists of a chamber of gas which can be made to ionize by the radiation and the resulting electric charge is collected through the use of a high voltage between the chamber wall and a thin wire in the center of the chamber. Ionized pairs of electrons and atoms are drawn to the opposite polarity, which will be the wall or the wire, depending how the voltage is applied, under the influence of the resulting electric field. This charge is then collected and amplified as a signal in a charge sensitive amplifier and then may be compared to a voltage threshold to determine whether a valid detection has been made. A disadvantage of an ionization chamber in a production environment is that the gas may leak out of the chamber or may break down and degrade through use and the chamber is usually quite large to achieve efficiency, making it difficult to apply to many environments. Moreover, the ionization chamber requires a very high voltage, typically in the range of 1,000 to 3,000 volts, and is also costly which further inhibits its widespread use as a thickness measuring device.

The scintillation detector, which is also known in the art to measure thickness, uses a material called a scintillator, which when it absorbs a unit of radiation becomes activated and will deactivate by giving off light in the ultraviolet range through the process of scintillation. This ultraviolet light is then detected by a quite sensitive light detector known as a photomultiplier tube. The photomultiplier tube is closely coupled to the scintillator material so that ultraviolet light will pass into it. A detection signal from a first photocathode within the photomultiplier tube is typically multiplied by a series of dynode stages which are successively more positive in charge than the preceding dynode stage. The multiplied signal is then taken as an output signal. A disadvantage of the scintillation detector is that it also uses lethal high voltages in the 1,000 to 3,000 volt range and in addition the photomultiplier tube characteristics change and drift with use and age and with fluctuations in the high voltage level. This type of a detector is also prone to breakage since it is an evacuated glass tube and because of its size, weight and cost this type of detector also cannot be used in many types of environments.

Semiconductor diode pasrticle detectors are also known. They consist of a p-type layer, an n-type layer and an optional intrinsic layer in the middle. This diode is sensitive to light and nuclear and atomic radiation when the same strikes the diode. Detectors of this type have been used for high resolution nuclear spectroscopy. The signal generated by this type of detector consists of the collection of charge released when the radiation loses energy in the sensitive region of the detector. This charge is collected across the p-type and n-type materials and is amplified with a high performance charge sensitive amplifier.

Typically these types of detection devices have a very small output signal. The output signal is so small that noise generated by thermal effects or impurities and imperfections in the silicon material will overcome the charge signal. For this reason, such detectors are typically operated at quite low temperatures, usually through the use of a liquid nitrogen coolant to arrive at adequate performance. While this type of detector could be used in laboratory research, it cannot be practically used in a production environment.

US-A-4301366 describes a thickness measuring apparatus in which a particle radiation detector receives particle radiation which has passed through the material whose thickness is to be determined. The detector output is amplified and filtered and applied to a comparator. The output of the comparator is processed to provide a signal representation of the thickness.

US-A-3016460 describes another known thickness measuring device and an article in Journal of Applied Physics, Vol. 53 No. 4, April 1982, pages 2975-2982 describes an X-ray detector which uses PIN diodes.

The object of the invention is to provide a thickness/density measuring apparatus with an improved detector arrangement and simple calibration facility.

According to the present invention there is provided a thickness/density measuring apparatus comprising:

a detector of x, $\gamma$ or particle radiation, such as beta rays for receiving said radiation,
a charge sensitive preamplifier coupled to the output of said detector;
an amplifier coupled to the output of said preamplifier;
a comparator coupled to the output of said amplifier for providing an output signal when the level of the output of said amplifier exceeds a set threshold, means responsive to the output of said comparator to provide a pulse rate signal, and means for converting the pulse rate signal to a signal representative of the thickness or density of material through which said radiation has passed,

characterised in that said detector is a PIN diode and said apparatus includes means for preventing light from impinging on said diode, but permitting said radiation to impinge on said diode, and wherein said apparatus further comprises means arranged to calibrate said apparatus for a thickness or density measurement using a single calibration sample, said calibrating means comprising means for entering an actual thickness or density value $(T_s, D_s)$ for said single calibration material sample, means for determining a sample pulse count rate $(C_s)$ for said material sample, means for storing the values $(C_s)$ and $(T_s, D_s)$, means for entering an indication of material type for a measured material, means for storing said entered material type, and means for storing a plurality of slope values (M) for various combinations of a sample pulse count rate $(C_s)$, and entered actual thickness or density value $(T_s, D_s)$ and an entered material type.

Embodiments of the present invention make particular use of a new type of silicon diode detector which contains a player, an n-layer and an intrinsic layer between them and which is operable at room temperature to detect radiation. The device, termed a PIN diode, is primarily intended for detection of laser and ultraviolet light, but it is known that this detector is also sensitive to nuclear and atomic radiation such as beta particles, X-rays and gamma rays.

The PIN diode is employed in a particular mechanical and circuit configuration to enhance its ability to detect particle radiation only. A light blocking window is used to screen the PIN diode from the effects of light radiation while the output signal therefrom is connected to a charge sensitive preamplifier. A high quality amplifier is then connected to the output of the preamplifier to develop a suitable signal which can be provided to a threshold detector, the output of which is sent to a pulse rate counter. The output of the pulse rate counter can be used together with pulse rate information derived during previous calibration use of the invention for a material of known thickness to determine a thickness measurement from a detected pulse rate when a material of unknown thickness is provided in the path of the radiation from a source to the PIN diode detector. Similar calibration and detection steps can be used to employ the invention as a density measuring device.

By using a PIN diode and associated small scale electronics package, a small compact detector can be provided which will allow it to be used in many applications that present generation thickness measuring devices based on the principle of particle radiation cannot approach. Typically, such a detector could be used in profile extrusion, blown film and blow molding, as well as the typical areas such as sheet and film extrusion. Moreover, since the measurement instrument of the invention requires only low voltage circuits, there is no safety hazard and associated heavily insulated power cables and connections are avoided thereby opening up new installation possibilites over present day equipment. Still further, the small size and compact solid state construction can provide high reliability and stability, while minimizing the possibility of damage of breakage of glass tubes or gas chambers typically associated with prior art devices. Fewer calibration operations and fewer repairs are also attendant the construction. Finally, because of its compact size, solid stage construction and use of relatively low cost components, the detector can be manufactured at lower cost than present generation thickness measurement systems.

Because of its low cost and compact construction, a plurality of measuring devices can be arrayed across an extruded film to measure the material thickness simultaneously across its width at discrete points. Because of the complexity and size of present generation equipment, this approach cannot now be achieved, and instead mechanical scanning of a thickness measuring device across the width of a sheet must be employed which has undesired complexity due to the mechanical scanning mechanism. Each of the plurality of thickness measuring devices provided in the array can then be used to control a respective section of processing machinery such as respective lip sections of an extrusion die to control the extruded product to desired specifications.

The invention will be described now by way of example only, with particular reference to the accompanying drawings. In the drawings:

Fig. 1 illustrates in electrical block and schematic form a first embodiment of the present invention;
Fig. 2 (not forming part of the present invention) illustrates in flow chart form a microprocessor program for a method of calibrating and measuring an unknown thickness using the embodiment shown in Fig. 1;
Fig. 3 illustrates in flow chart form a microprocessor program for a method of calibrating and measuring an unknown thickness in accordance with the invention;
Fig. 4 illustrates a modification of the Fig. 1 embodiment showing a different way of mounting a particle radiation detector;
Fig. 5 illustrates another modification of the Fig. 1 embodiment;
Fig. 6 illustrates a modification of the Fig. 4 embodiment;
Fig. 7 illustrates another modification of the Fig. 1 embodiment using a back-scatter radiation technique;

Fig. 8 illustrates a control system for an extrusion die utilizing the present invention;
Fig. 9 illustrates an array of thickness measuring detectors for use in a control system for controlling the thickness of an extruded film;
Fig. 10 (not forming part of the invention) illustrates in flow chart form a microprocessor program for a method of calibrating and measuring an unknown density using the embodiment shown in Fig. 1; and
Fig. 11 illustrates in flow chart form a microprocessor program for a method of calibrating and measuring an unknown density in accordance with the invention.

## Detailed Description of the Invention

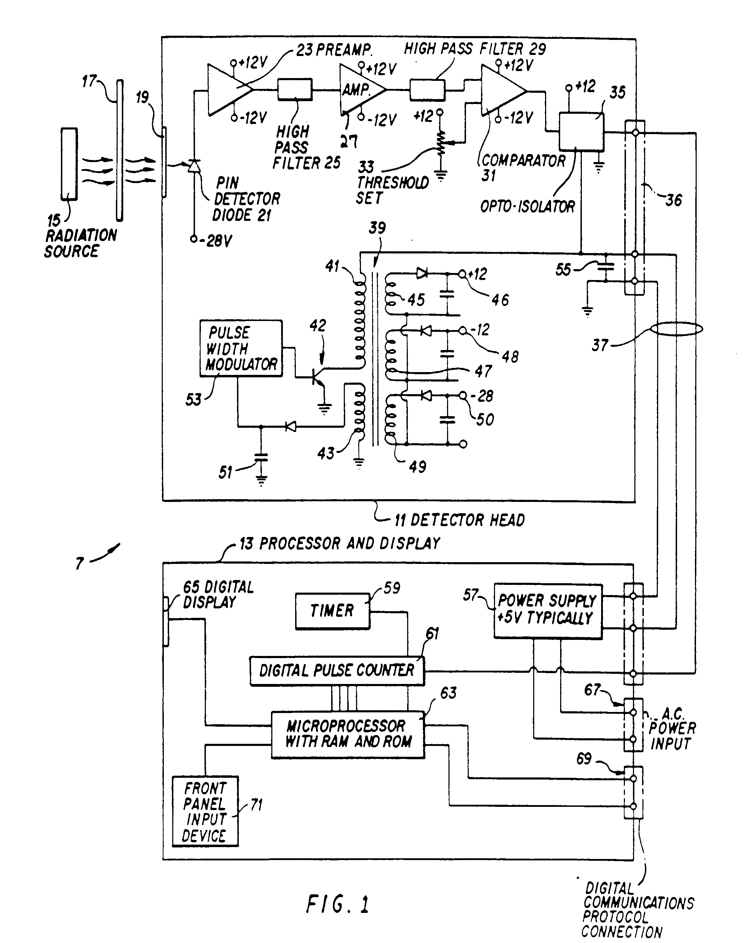

Fig. 1 illustrates the thickness/density measuring system of the invention in partly schematic, partly block diagram form. The thickness/density measuring system 7 is constructed in two component parts identified in Fig. 1 as detector head section 11 and processor and display section 13. These sections are interconnected by a low-voltage cable 37 and are described more fully below.

Detection head section 11 includes the detecting electronics for a thickness/density measurement, while processor and display section 13 contains the processing and display components for providing a digital display of a thickness/density measurement and/or an output signal representative of the thickness/density measurement which can be used for control purposes.

Turning now to the detector head section 11, it includes a window 19 in the housing which blocks entry of light radiation into the housing, but which permits particle radiation to pass therethrough. Suitable materials which can be used for window 19 include aluminized mylar®. Disposed behind window 19 and within housing 11 is a PIN diode 21 which is sensitive to radiation, both light radiation and particle radiation. As noted, window 19 is impervious to light radiation so that PIN detector 21 can only detect radiation such as X-rays, beta rays and gamma rays. This particle radiation is emitted by a radiation source 15 which is spaced from window 19. The material 17 to be measured is placed between the radiation source 15 and window 19.

The output of PIN diode 21 is connected to a charge sensitive preamplifier 23 the output of which is connected to a first high pass filter 25. The output of the high pass filter 25 is in turn passed to the input of a low noise operational amplifier 27, the output of which then passes through a second high pass filter 29. The output of the high pass filter 29 represents voltage pulses which correspond to charges which are detected by the PIN diode 21. The output of the high pass filter 29 is connected to the input of a comparator 31 which has a threshold set by resistive network 33 applied to another input thereof. The high pass filters block low frequency noise components while the threshold of comparator 31 is set so that miscellaneous noise which does not constitute a valid detection pulse will not affect the readings of the instrument. When the output of the high pass filter 29 exceeds the set threshold the comparator 31 provides an output pulse which is then fed to an opto-isolation and line driver circuit 35. The output of the isolation circuit 35 is then connected to a terminal strip on the detector head section 11 for connection with additional processing electronics in the digital processing and display section 13.

Before describing the digital processing and display section 13, the remainder of the circuitry provided in the detector head section 11 will be described. In order to provide operative power to the various electronic components within the detector head section 11, a power supply is built therein. Because the PIN diode requires different voltages than other electronic components within the detector head assembly, a multivoltage power supply is provided in the form of a transformer 39 having a plurality of primary windings 41 and 43 and a plurality of secondary windings 45, 47 and 49. The secondary windings are connected to respective diode/capacitor networks and provide the operating D.C. voltages required for the various circuit components within the detector head section 11. One side of primary winding 41 is connected to receive an unregulated input voltage applied to an input terminal strip 36 at the detector head section 11 and received from the digital processing and display section 13. This unregulated voltage supply is switched by a transistor switching device 42 under control of a pulse width modulator 53. Pulse width modulator 53 also receives a D.C. voltage input from a primary reference winding 43 through a diode capacitor network such that the pulse width modulator 53, network 51 and switching device 42 as well as the primary winding connections function to provide a regulated voltage output at the secondary terminals 46, 48 and 50.

An important aspect of the present invention is that the detector head section 11 can be made of very small size and the power supply for the detector head operates on low D.C. voltages which makes the detector head suitable for placement in many environments wherein high voltages and/or bulky and complex equipment cannot be provided.

The digital processing and display section 13 provides a low voltage power supply input to the detector head 11 and suppliers this via a power supply circuit 57 which receives an A.C. power input 67. Alternatively, a D.C. power source can be provided. The digital processing and display section 13 further includes a timer 59 which defines time windows during which a digital pulse counter 61 is enabled to count pulses which are received from the output of the opto-isolation circuit 35 in the detector head section 11. The pulse counter 61 is gated by the tinier 59 to establish a pulse rate counting circuit which provides a count pulse for a given period of time. The output of the digital pulse counter 61 is in turn gated into a microprocessor 63 which includes the usual ROM and RAM memory circuits for storing programs and data. A manually operable panel input service 71, containing a plurality of input switches or keys and input setting devices, is also provided for providing input information to the microprocessor 63. The microprocessor also provides as output signals a digital signal on terminals 69 representing a thickness or density measurement and an output signal to a digital display 65 provided within the digital processing and display section 13 also representing a thickness or density measurement.

At this point it should be noted that the first embodiment of the invention can be used for either a thickness or a density measurement, depending on the programming of microprocessor 63 as will be more fully described below.

Microprocessor 63 contains an algorithm for calculating a thickness or density measurement based on the count rate it receives from the digital pulse counter 61. It is first necessary to calibrate microprocessor 63 with reference data corresponding to reference data which indicates a known thickness or density and a measured count rate for that known thickness or density so that later measurements can be related to this calibration standard. The calibration for and measurement of an unknown thickness of a film will first be described.

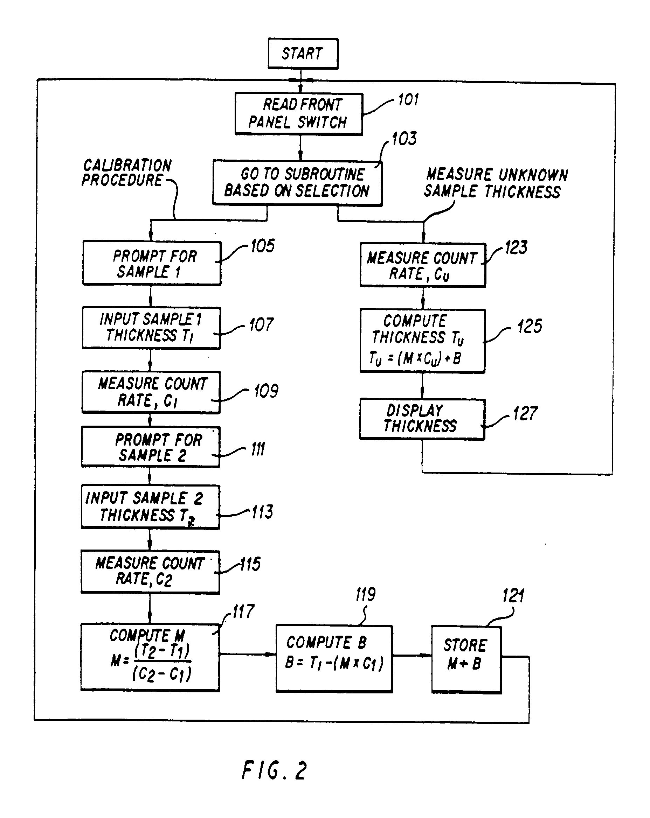

Fig. 2 illustrates in flow chart form the programming of microprocessor 63 which enables it both to be calibrated in the first instance and then to take thickness measurements thereafter. The operation depicted in Fig. is based on a two sample calibration technique. In a first step 101 of the processing, a switch on the front panel input device 71 of Fig. 1 is read to determine whether a calibration or a measuring operation is desired. In step 103 the microprocessor determines which type of processing is required. If a calibration procedure is selected by the front panel input devices 71, the microprocessor proceeds to step 105 where it displays on the digital display 65 a prompt to an operator instructing him to insert a reference sample into the measuring path between source 15 and window 19. In addition, the microprocessor further reads a thickness setting input device, e.g., digital value switches, on the front panel input device 71 to obtain a signal representing an actual thickness $T_1$ of this sample. Following step 107 the microprocessor proceeds to step 109 where it measures the count rate $C_1$, for the sample of known thickness in step 109. Following this the microprocessor activates the digital display 65 to prompt an operator to place a second sample of known thickness between the radiation source 15 and window 19. In addition, the microprocessor receives as an input on front panel input device 71 an entered thickness $T_2$ corresponding to the second sample. Thereafter, the microprocessor proceeds to step 115 and measures the count rate $C_2$ for the second sample and after this proceeds to step 117 where it computes a slope M value using the equation:

$$M = (T_2 - T_1)/(C_2 - C_1) \qquad (1)$$

Following this, the microprocessor proceeds to step 119 where it calculates an intercept value B as

$$B = T_1 - (M \times C_1) \qquad (2)$$

The slope value M and intercept value B are then stored in step 121 for later use by the instrument in calculating thickness of a sample of unknown thickness.

Returning to step 103, if an actual measurement is desired, the microprocessor proceeds from step 103 to 123 where it measures a count Cu rate for a sample of unknown thickness. Thereafter it computes an actual thickness $T_u$ using the equation

$$T_u = (M \times Cu) + B \qquad (3)$$

where M and B are the values previously obtained in the calibration step. This yields a thickness measurement which is then displayed on digital display 65 in step 127 or alternatively output to control equipment on lines 69 of the digital processing and display unit 13.

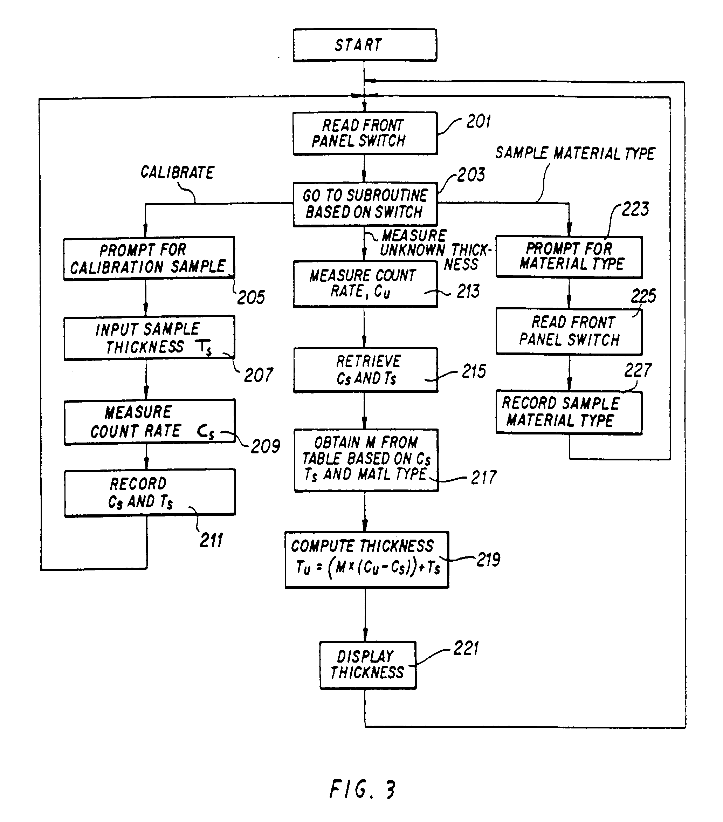

Fig. 3 shows an alternate thickness calibration and measurement program which can be used with the microprocessor 63. In this processing sequence, a single calibration sample is used to calibrate the instrument. In a first step 201 the front panel input device 71 is read to determine whether a calibration, a measurement, or a material type entry routine is to be executed. If the front panel switch at input device 71 indicates a calibration routine is desired, step 203 causes retrieval and execution of the calibration subroutine. The microprocessor thus proceeds to step 205 where it prompts an operator on the digital display 65 to insert a calibration sample between the radiation source 15 and window 19. In addition, a thickness $T_s$ of the material sample is input at step 207 on the front panel input device 71 and following this step the microprocessor proceeds to step 209 where it measures a count rate $C_s$. It then records the measured count rate $C_s$ and the inserted sample thickness $T_s$ in step 211 and proceeds back to step 201. With the Fig. 3 calibration technique, a material type entry, e. g., polyester, nylon, acrylic, etc. must also be made at input device 71. When the material type entry is indicated on the front panel input device 71, the microprocessor 63 senses this in step 203 and then branches to step 223 where it displays a prompt on the digital display 65 for an operator to enter, via the front panel input device 71, a material type which is used in the calibration. Material type as set in the front panel input device 71 is then read by the microprocessor 63 in step 225 recorded and stored in step 227. All the data required for the microprocessor now to calibrate itself for making an actual measurement of the material thickness is present. Thus, when the front panel input device 71 is now set for a measurement, step 203 executed by the microprocessor will cause a measurement routine to be started at step 213 where the count rate Cu of an unknown sample placed between the source 15 and window 19 is taken. After this, in step 215, the values $C_s$ and $T_s$ obtained during the calibration routine are retrieved following which, in step 217, an M value is obtained based on a table stored in the microprocessor interrelating the count values $C_s$, the set thickness $T_s$ and the inserted material type. This table is a stored lookup table and contains various values of M based on various combinations of values of $C_s$, $T_s$ and material type. Following step 217, where the value M is obtained from the lookup, a thickness computation is made in step 219 based on the formula

$$T_u = (M \times (C_u - C_s)) + T_s \qquad (4)$$

The thickness value is then displayed in step 221 on display 65 or output on lines 69 to further processing or control apparatus, following which the microprocessor proceeds back to the beginning of the program.

The calibration techniques described in Figs. 2 and 3 have their respective advantages and disadvantages. The calibration technique of Fig. 2 is straightforward and requires only operator entry of known thickness values, but requires two samples of different known thicknesses for calibration. While it provides a very good calibration of the instrument, the requirement of having two samples of close, but measurably different thickness, may be difficult at times and sometimes even impossible to achieve. Consequently, the Fig. 3 calibration technique, which is somewhat more cumbersome to execute, requires only one sample, has that as one benefit. The theory on which the calibration of Fig. 3 is based is that a particular count rate observed under any given circumstances will be effected by a number of factors including source strength, source to detector separation and geometry, losses in the detector window, dirt accumulation on the detector window, electronic sensitivity and threshold setting, and, of course, material composition and thickness. A single calibration sample observation will establish the net result of the combination of these parameters at a particular point in time. In production use, the only variable of practical concern is the unknown material thickness for a reasonable period of time. A reasonable period of time is established by such factors as a half life of a radioactive source which typically would be quite long compared to a production period, the drift rate of the electronics, and the rate of accumulation of dirt on the window. The Fig. 3 calibration technique is based on the fact that once a count rate has been established for a given thickness of a type of material under a certain condition of source strength, source to detector separation etc., the variation and count rate with material thickness represented by M in Equation 4 above, can be predetermined and stored permanently in the device for use during production measurements. Since M will be somewhat dependent on the nominal count rate and type of material being measured, a number of values of M can he predetermined and stored in the microprocessor 63 and an M can be selected by microprocessor 63 which most closely matches the operating conditions. The material type is thus operator selectable through the front panel input device 71 and the nominal count rate can be taken during a calibration sample count so that the microprocessor 63 can then make appropriate selection of the value M. To determine the initial values of M which can be permanently stored in the microprocessor 63 repeated application of Equation 1 above can be used over a range of material types and thicknesses. These results can be arranged in a tabular form so that when a particular calibration sample thickness and material type are entered and the count rate observed, the value of M which most closely matches these conditions can be selected for use.

In summary, the calibration technique illustrated in Fig. 3 has the advantage that a single sample of production material, manually measured for thickness, can be used as the calibration sample and to establish the nominal count rate of production for the algorithm to select a proper value of M for later thickness measurements. A single step calibration procedure is thus provided which is more readily used by unskilled operators.

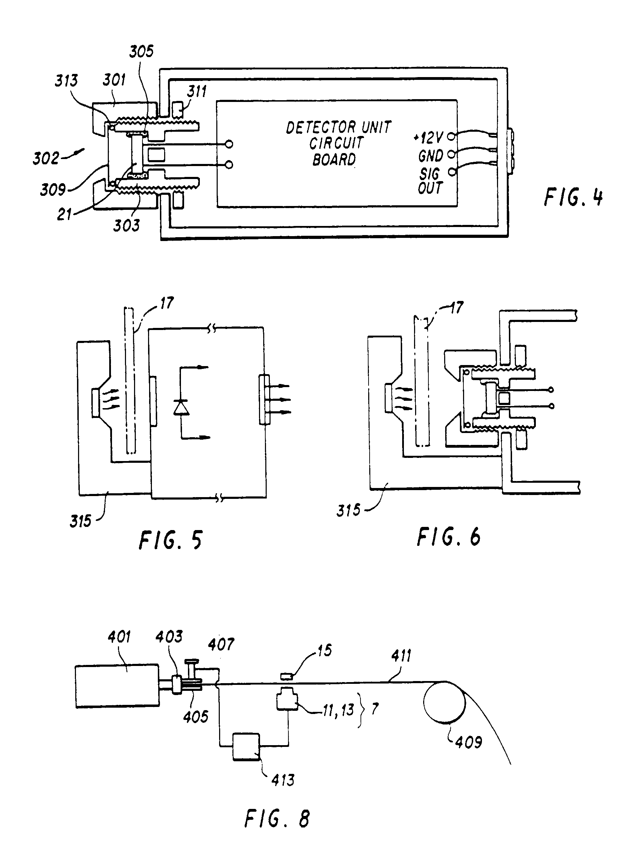

Fig. 4 illustrates a modification of the detector head 11 illustrated in Fig. 1. In this embodiment, an easily removable detector head assembly is provided which can be readily fixed to production machinery. The PIN diode 21 is shown bonded by an Epoxy 305 or similar adhesive to the inner periphery of an exteriorly threaded sleeve 303. An aluminized mylar® entrance window in the form of a film 309 is provided at one end of the threaded sleeve 303 with an O-ring seal 313 being provided between the mylar® window and an end of threaded sleeve 303. A locking cap 301 is threaded onto the outer periphery of threaded sleeve 303 which serves to hold the mylar film 309 in place and which also serves to provide a backstop which limits the amount of travel of the threaded sleeve into an opening in the section 11. Cap 301 includes an opening 302 at a front side thereof at a position corresponding to the location of the mylar window 309. A backing nut 311 is screwed onto the threaded shaft 303 behind a wall of the detector head housing 11 to hold the entire detector head in place. The leads of the PIN diode 21 are then affixed to an electronic circuit board containing the remainder of the electronics of the detector head assembly shown in Fig. 1. The design of the housing illustrated in Fig. 4 is particularly advantageous as it allows ready replacement of the actual detector head itself, including the PIN diode, without requiring a complete disassembly of the components within housing 13.

The aluminized mylar window 19 and 309 illustrated in Figs. 1 and 4 can be formed of a mylar film having a thickness of 0.5 to 5.0 mils. The window can also be made of a thin aluminum or stainless steel foil. In addition, the PIN diode illustrated in Figs. 1 and 4 is a windowless construction wherein protective layers covering the active PIN areas are not provided by the manufacturer to maximize radiation sensitivity. Thus, the mylar® window 19 or 309 provides the principal form of protection for PIN diode 21. Mylar® window 309 of Fig. 4 contains the same aluminized construction as Fig. 1 which blocks light radiation from striking the PIN diode 21, thereby improving reliability of detection of particle radiation.

In the embodiments of the invention thus described, the radiation source, which may emit gamma rays, X-rays, or beta rays, is fixedly spaced from the detector head section 11 by being mounted on a separate support. It is also possible to provide an integral support on the detector head section 11 for the detector source which would then provide a fixed gap between the source and PIN detector within which a material whose thickness is to be measured can be placed. This embodiment is illustrated in greater detail in Figs. 5 and 6 which respectively correspond to the embodiments of Figs. 1 and 4, but with an extension arm 315 integrally connected to section 11 and extending therefrom in a L-shaDe fashion to provide a gap between the radiation source and radiation detector. With this integral construction, it is possible to construct the entire detector unit as a simple portable assembly. Moreover, since the detector unit operates on low voltages, it is further possible to incorporate all of the electronics including those of the digital processing and unit provided in section 13 of Fig. 1 into a single housing which is readily portable to different environments of use.

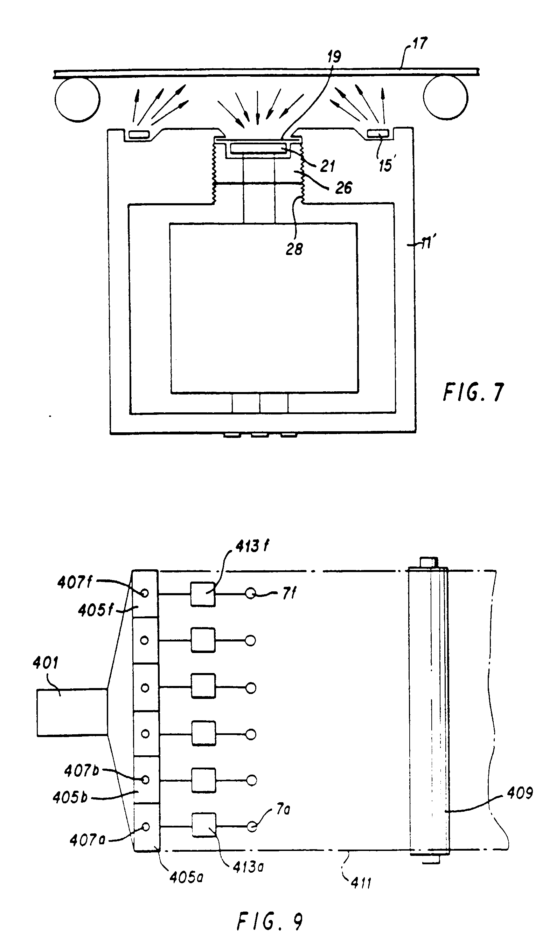

Fig. 7 illustrates a modification of the invention wherein the detector head section 11' is used in a backscatter radiation detection technique. In this embodiment, the PIN diode 21 is mounted within a relatively thick material portion of one end of the section 11. In addition, the radiation source 15' is also mounted in the same end of the housing but the housing is configured so that most, if not all, of the radiation from source 15 projects outwardly of the housing and not directly toward the PIN diode 21. As a consequence, a material can be stretched across the one end of the section 11 in front of the radiation source 15 and PIN diode 21, and backscatter radiation can be detected from the source by the PIN diode. The amount of backscattering which occurs will be dependent on material thickness and thus the instrument can be caliabrated for radiation count based on backscatter using either of the calibration techniques described above with respect to Figs. 2 and 3. In the Fig. 7 embodiment, the PIN diode is mounted in a PIN diode holder 26 which has external screw threads which screw with a threaded bore 28 provided in one end of the detector head section 11.

The only difference between the microprocessor 63 programming for calculating a thickness value when the backscatter approach is used is that the numerical sign of the slope constant M will evaluate to be positive when the calibration procedure is followed. This is due to the fact that the count rate increases for increasing material thickness in the backscatter mode while it decreases for increasing material thickness in a transmission mode.

Figs. 8 and 9 illustrate a control system with which the thickness measuring device 7 of the invention can be particularly suitably employed. The control system is used to control sections of die lips of a film extrusion system. Fig. 8 is a side view of the system which includes an extruder barrel 401 supplying a plasticized material to a die 403 for extruding a thin sheet of plastic material. Die 403 includes die lips 405 at the outlet end thereof which are adjustable with respect to the gap defined by the lips to thereby adjust the thickness of the material leaving the die. Typically, a bolt 407 is used to adjust the die lips with manual operation of the bolt being used to effect a coarse adjustment and with a system providing a fine adjustment of the gap between the die lips. In many instances, the fine adjustment is provided by actually heating the bolt 407 to provide fine adjustment of the die gap. This heating is controlled by a control system 413 which includes a typical PID (proportional, integral, derivative) controller which in turn is connected to the output of the thickness measuring device 7 illustrated in Fig. 1, particularly to the output provided at terminals 69. Film 411 which passes from the die lips is taken over a haul-off roller 409. In order to measure thickness, a radiation source 15 is provided on one side of the film and the detector, including housing sections 11 and 13 are provided on the other side of the film. Alternatively, a backscatter technique may be employed as illustrated in Fig. 7. In either event, the output signal of the thickness measuring device is applied to the PID controller 413 to in turn control the heating of bolt 407 which finely controls the gap of die lips 405. As illustrated in Fig. 9, for a sheet of substantial width, the die lips 405 are divided into a plurality of die lip sections 405a... 405f, with each being controlled by respective die bolts 407a...407f and with the heating of each die bolt being in turn controlled by a respective PID controller 413a... 413f. The inputs to each of these controllers are respectively derived from individual thickness measuring devices 7a...7f constructed as described above. With this arrangement, each measuring device 7 measures a portion of the width-wise extent of the film 411 and in turn controls individually its own heater bolt 407 so that the thickness of each section of the width-wise dimension of the film is individually and separately controlled.

Thus far, the invention has principally been described with reference to thickness measurements. However, it is also possible to use the same apparatus for measuring density. To allow density measurement, three conditions must occur, the thickness of a material placed between the source and detector must remain constant, or the material completely fills the volume between the source and detector as in a fluid medium, or the thickness is known and fed into an algorithm by an external thickness measuring device which can then calculate a density from the measured thickness and known material characteristics.

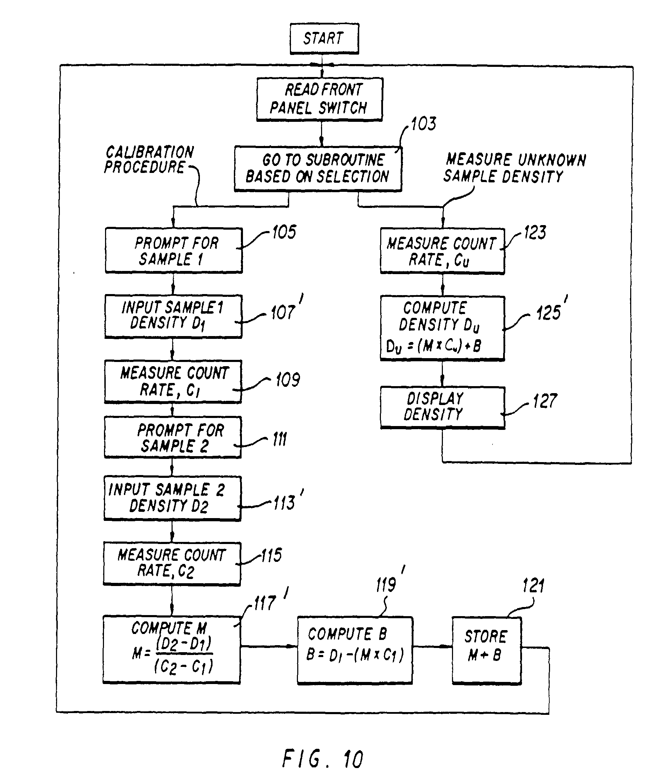

Fig. 10 shows a modification of the Fig. 2 flow chart to permit use of the Fig. 1 apparatus for a density measurement. As shown therein, step 107 is modified as new step 107', step 113 is modified as new step 113', and steps 117, 119 and 125 are all modified as new steps 117', 119' and 125'.

Without describing again all of the steps illustrated in Fig. 2 which are common in Fig. 10, those steps which are common have the same reference numbers. Those steps which are changed are designated by a prime (') symbol. In step 107' of Fig. 10, a density $D_1$ is entered for a first material sample while in step 113' a density $D_2$ is entered for a second material sample. In step 117', the slope M is computed using the formula

$$M = (D_2 - D_1)/(C_2 - C_1) \qquad (5)$$

In step 119', the intercept is computed as

$$B = D_1 - (M \times C_1) \qquad (6)$$

With the now stored calibration values of M and B, a density measurement can then be carried out, with the density computation made in step 125' as

$$D_u = (M \times C_u) + B \qquad (7)$$

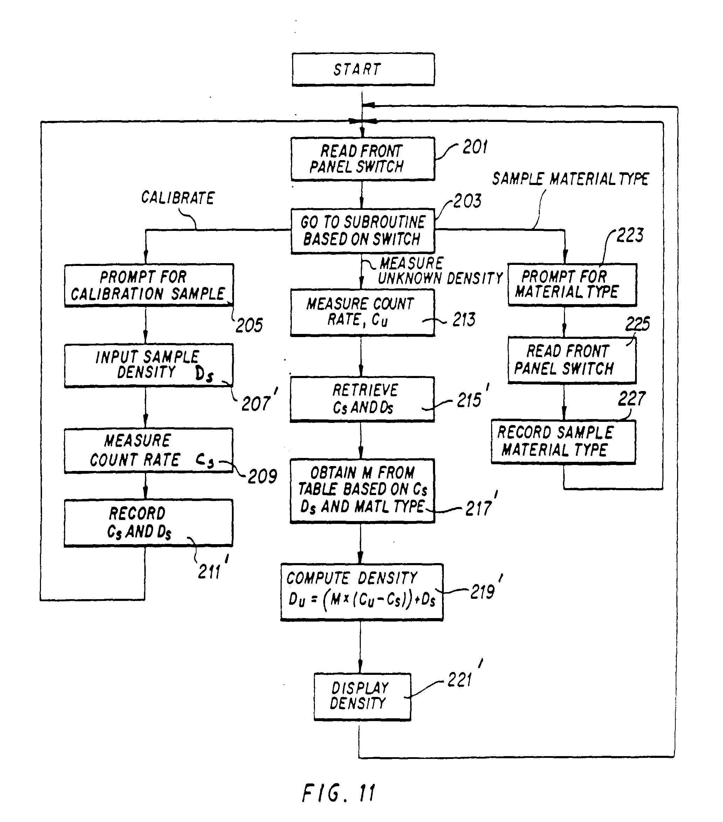

Fig. 11 illustrates the modifications needed for the Fig. 3 flow chart to attain a density measurement.

In the calibration technique for density measurement illustrated in Fig. 11, an input sample density is input to the system in step 207' and this together with a detected count rate $C_s$ for the sample is recorded at step 211'. A sample material type is also entered and stored at step 227. Finally, to calculate a density measurement, a measured count rate Cu at step 213 is first taken following which values $C_s$ and $D_s$ entered during the calibration sequence are used to obtain a slope value M from a table based on $C_s$, $D_s$ and material type. From this, a density measurement can then be calculated using subroutine represented by steps 213, 215, 217', 219' and 221' as follows. A count rate is measured at step 213 following which the value $C_s$ and $D_s$ are retrieved in step 215'. From this the value of M is obtained from a stored table based on the values of $C_s$s, $D_s$ and material type. In step 219' the density $D_u$ is computed from the formula

$$D_u = (M \times (C_u - C_s)) + D_s \qquad (8)$$

The density value is then displayed on digital display 65 or sent to output terminals 69 in step 221'.

It is also possible to calculate a density when a thickness of a sample is known or measured from the equation

$$D_u = T_s/T_u \times ((M(C_u - C_s)) + D_s) \qquad (9)$$

where $D_s$ is a density of a calibration sample, M is the slope factor calculated in the manner described in steps 213...221' of Fig. 11, $T_u$ is a thickness of a sample in front of the detector and $T_s$ is a thickness of a density standard.

It is also noted that the apparatus of the invention can also be used as a material flaw or void detection apparatus since flaws and voids can be recognized by a change in a density value.

As is apparent from the foregoing description, the present invention provides a compact thickness/density measuring apparatus which can be used in a wide range of environments, particularly environments where high voltages and/or complex and bulky structures cannot be used. The measurement instrument is compact and easily transportable and can be configured as a portable unit.

While various embodiments of the present invention have been described and illustrated in connection with the drawings, it should be apparent that many modifications can be made to the invention as described, without departing from the scope of the invention. Accordingly, the invention is not limited by the foregoing description but is only limited by the scope of the claims appended hereto.

## Claims

1. A thickness/density measuring apparatus comprising:

   a detector of x, $\gamma$ or particle radiation, such as beta rays (21) for receiving said radiation,
   a charge sensitive preamplifier (23) coupled to the output of said detector;
   an amplifier (27) coupled to the output of said preamplifier;
   a comparator (31) coupled to the output of said amplifier for providing an output signal when the level of the output of said amplifier exceeds a set threshold,
   means (13) responsive to the output of said comparator to provide a pulse rate signal, and
   means for converting the pulse rate signal to a signal representative of the thickness or density of material through which said radiation has passed,

   characterised in that said detector (21) is a PIN diode and said apparatus includes means (19) for preventing light from impinging on said diode, but permitting said radiation to impinge on said diode, and wherein said apparatus further comprises means arranged to calibrate said apparatus for a thickness or density measurement using a single calibration sample, said calibrating means comprising means for entering an actual thickness or density value $(T_s, D_s)$ for said single calibration material sample, means for determining a sample pulse count rate $(C_s)$ for said material sample, means for storing the values $(C_s)$ and $(T_s, D_s)$, means for entering an indication of material type for a measured material, means for storing said entered material type, and means for storing a plurality of slope values (M) for various combinations of a sample pulse count rate $(C_s)$, an entered actual thickness or density value $(T_s, D_s)$ and an entered material type.

2. A thickness/density measuring apparatus according to claim 1, wherein said converting means comprises means for computing a thickness value in accordance with the following:

$$T_u = (M \times (C_u - C_s)) + T_s$$

   where $T_u$ is the unknown thickness value, $C_u$ is the pulse rate signal, $T_s$ is the actual thickness of a material sample used for calibration, $C_s$ is the pulse count rate for said material sample, and M is a slope value selected from said plurality of slope values which correspond to an entered $T_s$ value, a determined $C_s$ value, and an entered material type.

3. A thickness/density measuring apparatus according to any one of claims 1 to and 2, further comprising a radiation source (15) spaced from said detector.

4. A thickness/density measuring apparatus according to claim 3, wherein said detector is mounted in a housing and source (15) is mounted on an arm (315) extending from said housing at a position to oppose said detector whereby a gap is formed between said source and detector for accommodating a material to be measured.

5. A thickness/density measuring apparatus according to claim 3, further comprising means for mounting said radiation source (15) relative to said detector (21) such that said radiation detected by said detector is back-scattered by said material to be measured.

6. A thickness/density measuring apparatus according to any preceding claim, wherein said conductive layer is a window and is formed of an aluminized mylar.

7. A thickness/density measuring apparatus according to any preceding claim, further comprising a first high pass filter (25) connected between said preamplifier (23) and amplifier (27).

8. A thickness/density measuring apparatus according to claim 7, further comprising a second high pass filter (29) connected between said amplifier (27) and comparator (31).

9. A thickness/density measuring apparatus according to any one of claims 2 to 8, further comprising an opto-isolator (35) connected between said comparator (31) and said means providing a pulse rate signal.

10. A thickness/density measuring apparatus according to claim 1, wherein said PIN diode (21) contains no protective layers between said preventing means (19) and radiation sensitive areas thereof.

11. A thickness/density measuring apparatus according to claim 1, wherein said means (13) includes a pulse rate counter (61) coupled to the output of said comparator means and means responsive to the output of said pulse rate counter for producing said signal.

12. A thickness/density measuring apparatus according to claim 11, wherein said means (13) includes a microprocessor for converting a pulse count rate into said signal.

13. A thickness/density measuring device according to any preceding claim, further comprising a power supply for supplying operating power to said diode, preamplifier, amplifier and comparator, said power supply comprising means for providing a first unregulated D.C. voltage of a first value and means for producing from said unregulated D.C. voltage a plurality of regulated D.C. voltages.

14. A thickness/density measuring apparatus according to claim 13, wherein said means for producing a plurality of regulated D.C. voltage comprises a transformer (39) for receiving said unregulated D. C. voltage at a first primary winding (41) thereof and for supplying said regulated D.C. voltages at a plurality of second windings (45, 57, 59), means (42) for switching the current through said first primary winding, a pulse width modulator (53) for operating said switching means, and means for coupling said pulse width modulator to a second primary winding of said transformer.

**Patentansprüche**

1. Dicken-/Dichtemeßvorrichtung mit

   - einer Erfassungseinrichtung (21) für Röntgen-, $\gamma$- bzw. Teilchenstrahlung, beispielsweise $\beta$-Strahlen,
   - einem mit dem Ausgang der Erfassungseinrichtung gekoppelten ladungsempfindlichen Vorverstärker (23),
   - einem mit dem Ausgang des Vorverstärkers gekoppelten Verstärker (27),
   - einem mit dem Ausgang des genannten Verstärkers gekoppelten Komparator (31) zur Abgabe eines Ausgangssignals, wenn der Pegel des Ausgangs des Verstärkers eine bestimmte Schwelle überschreitet,
   - einer auf den Ausgangswert des Komparators ansprechenden Einrichtung (13) zur Abgabe eines Pulsratensignals, sowie einer Einrichtung zur Umwandlung des Pulsratensignals in ein Signal, das die Dicke bzw. die Dichte des Materials repräsentiert, durch das die Strahlung hindurchgegangen ist,

   dadurch **gekennzeichnet**, daß die Erfassungseinrichtung (21) eine PIN-Diode ist und die genannte Vorrichtung Einrichtungen (19) zum Verhindern von Lichteinfall auf die Diode aufweist, während ein Auftreffen der Strahlung auf diese Diode ermöglicht wird, wobei die Vorrichtung weiter Einrichtungen aufweist, die vorgesehen sind, um die Vorrichtung im Hinblick auf die Dicken- bzw. Dichtemessung unter Verwendung eines einzigen Kalibrierungsmusters zu kalibrieren, und wobei die Kalibrierungseinrichtung Einrichtungen für die Eingabe eines tatsächlichen Dicken- oder Dichtewerts ($T_s$, $D_s$) für die genannte Kalibrierungsmaterialprobe aufweist, ferner Einrichtungen zur Bestimmung einer Probenpulszählrate ($C_s$) für die genannte Materialprobe, Einrichtungen zur Speicherung der Werte ($C_s$) und ($T_s$, $D_s$), Einrichtungen zum Eingeben einer Angabe über die Materialart für ein gemessenes Material, Einrichtungen zur Speicherung der eingegebenen Materialart sowie Einrichtungen zur Speicherung mehrerer Steigungswerte (M) für verschiedene Kombinationen einer Probenpulszählrate ($C_s$), eines eingegebenen tatsächlichen Dicken- oder Dichtewertes ($T_s$, $D_s$) sowie einer eingegebenen Materialart.

2. Dicken-/Dichtemessvorrichtung nach Anspruch 1, bei der die Umwandlungseinrichtung eine Einrichtung zur Berechnung des Dickenwerts gemäß der folgenden Gleichung umfaßt:

$$T_u = (M \times (C_u - C_s)) + T_s$$

wobei $T_u$ der unbekannte Dickenwert, $C_u$ das Pulsratensignal, $T_s$ die tatsächliche Dicke einer für die Kalibrierung verwendeten Materialprobe, $C_s$ die Pulszählrate für die Materialprobe und M ein Steigungswert ist, der aus einer Mehrzahl von Steigungswerten ausgewählt ist, die einem eingegebenen $T_s$-Wert, einem vorbestimmten $C_s$-Wert und einer eingegebenen Materialart entsprechen.

3. Dicken-/Dichtemessvorrichtung nach einem der Ansprüche 1 und 2, ferner eine von der Erfassungseinrichtung beabstandete Strahlungsquelle (15) aufweisend.

4. Dicken-/Dichtemessvorrichtung nach Anspruch 3, bei der die Erfassungseinrichtung in einem Gehäuse untergebracht ist und die Quelle (15) an einem von dem Gehäuse ausgehenden Arm (315) an einer Position befestigt ist, in der er der Erfassungseinrichtung gegenüberliegt, wodurch zwischen der Quelle und der Erfassungseinrichtung ein Spalt für die Aufnahme des zu messenden Materials gebildet wird.

5. Dicken-/Dichtemessvorrichtung nach Anspruch 3, ferner eine Einrichtung zur Befestigung der Strahlungsquelle (15) in Relation zu der Erfassungseinrichtung (21) umfassend, dergestalt, daß die von der Erfassungseinrichtung erfaßte Strahlung von dem zu messenden Material rückgestreut ist.

6. Dicken-/Dichtemessvorrichtung nach einem der vorangehenden Ansprüche, bei der die leitende Schicht ein Fenster ist und aus einem aluminiumbedampften Mylar gebildet ist.

7. Dicken-/Dichtemessvorrichtung nach einem der vorangehenden Ansprüche, ferner einen ersten Hochpassfilter (25) umfassend, der zwischen dem Vorverstärker (23) und dem Verstärker (27) angeschlossen ist.

8. Dicken-/Dichtemessvorrichtung nach Anspruch 7, weiter einen zweiten Hochpassfilter (29) umfassend, der zwischen dem Verstärker (27) und dem Komparator (31) angeschlossen ist.

9. Dicken-/Dichtemessvorrichtung nach irgendeinem der Ansprüche 2 bis 8, ferner einen optischen Isolator (35) aufweisend, der zwischen dem Komparator (31) und der Einrichtung vorgesehen ist, die das Pulsratensignal abgibt.

10. Dicken-/Dichtemessvorrichtung nach Anspruch 1, bei der die PIN-Diode (21) keine Schutzschichten zwischen der Verhinderungseinrichtung (19) und deren strahlungsempfindlichen Bereichen aufweist.

11. Dicken-/Dichtemessvorrichtung nach Anspruch 1, bei der die Einrichtung (13) einen Pulsratenzähler (61), der mit dem Ausgang der Komparatoreinrichtung gekoppelt ist, sowie eine auf den Ausgangswert des Pulsratenzählers ansprechende Einrichtung zur Erzeugung des genannten Signals aufweist.

12. Dicken-/Dichtemessvorrichtung nach Anspruch 11, bei der die Einrichtung (13) einen Mikroprozessor zur Umwandlung einer Pulszählrate in das genannte Signal enthält.

13. Dicken-/Dichtemessvorrichtung nach einem der vorangehenden Ansprüche, ferner eine Stromversorgung enthaltend, um die genannte Diode, den Vorverstärker, den Verstärker und den Komparator mit Strom zu versorgen, wobei die Stromversorgung eine Einrichtung für die Lieferung einer ersten ungeregelten Gleichspannung eines ersten Wertes und eine Einrichtung für die Erzeugung mehrerer geregelter Gleichspannungen aus dieser ungeregelten Gleichspannung aufweist.

14. Dicken-/Dichtemessvorrichtung nach Anspruch 13, bei der die Einrichtung zur Erzeugung mehrerer geregelter Gleichspannungen einen Transformator (39) zur Aufnahme der ungeregelten Gleichspannung an einer ersten Primärwicklung (41) von diesem sowie zum Liefern dieser geregelten Gleichspannungen an mehreren zweiten Wicklungen (45, 57, 59), Einrichtungen (42) zum Schalten des Stroms durch die erste Wicklung, einen Pulsbreitenmodulator (53) für die Betätigung der Schalteinrichtung sowie Einrichtungen zur Kopplung des Pulsbreitenmodulators an eine zweite Primärwicklung des Transformators aufweist.

**Revendications**

1. Appareil de mesure d'épaisseur/densité, comprenant:

   un détecteur (21) de rayons X, $\gamma$ ou particulaires, tel que des rayons $\beta$, pour recevoir les rayons,
   un préamplificateur (23) sensible à une charge, raccordé à la sortie du détecteur,
   un amplificateur (27) raccordé à la sortie du préamplificateur,
   un comparateur (31) raccordé à la sortie de l'amplificateur pour procurer un signal de sortie lorsque le niveau de la sortie de l'amplificateur dépasse un seuil réglé,

des moyens (13) qui répondent à la sortie du comparateur afin de procurer un signal de taux d'impulsion, et des moyens pour transformer le signal de taux d'impulsion en un signal représentant l'épaisseur ou la densité d'une matière à travers laquelle les rayons sont passés,

caractérisé en ce que le détecteur (21) est une diode PIN et en ce que l'appareil comprend des moyens (19) pour empêcher que de la lumière atteigne cette diode mais pour permettre que les rayons atteignent la diode, et en ce que l'appareil comprend de plus des moyens agencés pour calibrer l'appareil pour une épaisseur ou densité en utilisant une mesure d'un unique échantillon de calibrage, les moyens de calibrage comprenant des moyens pour introduire une valeur d'épaisseur ou de densité réelle ($T_s$, $D_s$) pour l'unique échantillon de matière de calibrage, des moyens pour déterminer un taux de compte d'impulsions d'échantillon ($C_s$) pour l'échantillon de matière, des moyens pour mémoriser les valeurs ($C_s$) et ($T_s$, $D_s$), des moyens pour introduire une indication d'un type de matière pour une matière mesurée, des moyens pour mémoriser le type de matière introduit et des moyens pour mémoriser une pluralité de valeurs de pente (M) pour différentes combinaisons d'un taux de compte ($C_s$) d'impulsions d'échantillon, d'une valeur réelle introduite d'épaisseur ou de densité ($T_s$, $D_s$) et d'un type de matière introduit.

2. Appareil de mesure d'épaisseur/densité suivant la revendication 1, caractérisé en ce que les moyens de transformation comprennent des moyens pour calculer une valeur d'épaisseur suivant ce qui suit :

$$T_u = (M \times (C_u - C_s)) + T_s$$

$T_u$ étant la valeur d'épaisseur inconnue, $C_u$ étant le signal de taux d'impulsion, $T_s$, étant l'épaisseur réelle d'un échantillon de matière utilisé pour le calibrage, $C_s$ étant le taux de compte d'impulsions pour l'échantillon de matière, M étant une valeur de pente sélectionnée parmi une pluralité de valeurs de pente qui correspondent à une valeur $T_s$ introduite, une valeur $C_s$ déterminée et un type de matière introduit.

3. Appareil de mesure d'épaisseur/densité suivant l'une ou l'autre des revendications 1 et 2, caractérisé en ce qu'il comprend en outre une source de rayons (15) écartée du détecteur.

4. Appareil de mesure d'épaisseur/densité suivant la revendication 3, caractérisé en ce que le détecteur est monté dans un boîtier et la source (15) est montée sur un bras (315) qui s'étend depuis le boîtier, à une position pour être opposée au détecteur, un intervalle étant formé par cela entre la source et le détecteur afin de recevoir une matière à mesurer.

5. Appareil de mesure d'épaisseur/densité suivant la revendication 3, caractérisé en ce qu'il comprend en outre des moyens pour supporter la source de rayons (15) par rapport au détecteur de façon à ce que les rayons détectés par le détecteur subissent une rétrodiffusion de la part de la matière à mesurer.

6. Appareil de mesure d'épaisseur/densité suivant l'une quelconque des revendications précédentes, caractérisé en ce que la couche conductrice est une fenêtre et est formée par un mylar aluminé.

7. Appareil de mesure d'épaisseur/densité suivant l'une quelconque des revendications précédentes, caractérisé en ce qu'il comprend en outre un premier filtre passe haut (25) raccordé entre le préamplificateur (23) et l'amplificateur (27).

8. Appareil de mesure d'épaisseur/densité suivant la revendication 7, caractérisé en ce qu'il comprend en outre un second filtre passe haut (29) raccordé entre l'amplificateur (27) et le comparateur (31).

9. Appareil de mesure d'épaisseur/densité suivant l'une quelconque des revendications 2 à 8, caractérisé en ce qu'il comprend en outre un coupleur optique (35) raccordé entre le comparateur (31) et les moyens qui procurent un signal de taux d'impulsion.

10. Appareil de mesure d'épaisseur/densité suivant la revendication 1, caractérisé en ce que la diode PIN (21) ne contient pas de couche protectrice entre les moyens d'empêchement (19) et des zones de ceux-ci, sensibles à des rayons.

11. Appareil de mesure d'épaisseur/densité suivant la revendication 1, caractérisé en ce que les moyens (13) comprennent un compteur de taux d'impulsion (61) raccordé à la sortie des moyens de comparaison, et des moyens qui répondent à la sortie du compteur de taux d'impulsion en vue de produire ledit signal.

12. Appareil de mesure d'épaisseur/densité suivant la revendication 11, caractérisé en ce que les moyens (13) comprennent un microprocesseur pour transformer un taux de compte d'impulsions en signal susdit.

13. Dispositif de mesure d'épaisseur/densité suivant l'une quelconque des revendications précédentes, caractérisé en ce qu'il comprend en outre une alimentation électrique pour fournir une énergie fonctionnelle à la diode, au préamplificateur, à l'amplificateur et au comparateur, l'alimentation électrique comprenant des moyens pour fournir une première tension continue non régulée d'une première valeur et des moyens pour produire à partir de cette tension continue non régulée une pluralité de tensions continues régulées.

14. Appareil de mesure d'épaisseur/densité suivant la revendication 13, caractérisé en ce que les moyens pour produire une pluralité de tensions continues régulées comprennent un transformateur (39) pour recevoir ladite tension continue non régulée à un premier enroulement primaire (41) de celui-ci et pour fournir les tensions continues régulées à l'endroit d'une pluralité de seconds enroulements (45, 57, 59), des moyens (42) pour commuter le courant à travers le premier enroulement primaire, un moduleur de largeur d'impulsion (53) pour faire fonctionner les moyens de commutation, et des moyens pour raccorder le moduleur de largeur d'impulsion à un second enroulement primaire du transformateur.

FIG. 1

START

READ FRONT
PANEL SWITCH — 101

GO TO SUBROUTINE
BASED ON SELECTION — 103

CALIBRATION
PROCEDURE

MEASURE UNKNOWN
SAMPLE THICKNESS

PROMPT FOR
SAMPLE 1 — 105

MEASURE COUNT
RATE, $C_u$ — 123

INPUT SAMPLE 1
THICKNESS $T_1$ — 107

COMPUTE
THICKNESS $T_u$
$T_u = (M \times C_u) + B$ — 125

MEASURE COUNT
RATE, $C_1$ — 109

DISPLAY
THICKNESS — 127

PROMPT FOR
SAMPLE 2 — 111

INPUT SAMPLE 2
THICKNESS $T_2$ — 113

MEASURE COUNT
RATE, $C2$ — 115

COMPUTE M
$M = \dfrac{(T_2 - T_1)}{(C_2 - C_1)}$ — 117

COMPUTE B
$B = T_1 - (M \times C_1)$ — 119

STORE
M + B — 121

F I G. 2

FIG. 3

FIG. 4

FIG. 5

FIG. 6

FIG. 8

FIG. 7

FIG. 9

FIG. 10

FIG. 11